# EUROPEAN PATENT APPLICATION

(11) **EP 4 491 154 A2**
(43) Date of publication of application: **15.01.2025**
(21) Application number: 24178261.4
(22) Date of filing: 27.05.2024
(51) Int. Cl.: A61F 2/24

(54) **CAPTURE DEVICES FOR CONTROLLED RELEASE OF PROSTHETIC VALVE DEVICES**

(30) Priority: 20.06.2023 US 202363509192 P; 06.03.2024 US 202418597198
(71) Applicant: Medtronic, Inc., Minneapolis, Minnesota 55432 (US)
(72) Inventor: OWENSON, Caitlin M., Santa Rosa (US); GROSSMAN, David A., Santa Rosa (US); STEINER, Jacob A., Santa Rosa (US)
(74) Representative: Zimmermann & Partner Patentanwälte mbB

(57) **Abstract**

A delivery system includes an outer shaft including a capsule at a distal portion thereof configured to restrain a prosthetic device, an inner shaft disposed within the outer shaft, and a capture device disposed between the outer shaft and the inner shaft. The capture device is configured to retain a portion of the prosthetic device. The capture device includes: a body including a central passage, a plurality of radial openings extending from the central passage radially outward to an outer surface of the body, and a plurality of slots in the outer surface of the body; and a plurality of capture loops, each capture loop extending radially outward through a corresponding radial opening, each capture loop configured to extend through a portion of the prosthetic device, and a distal end of each capture loop configured to be releasably restrained in a corresponding slot of the plurality of slots.

## Description

### CROSS-REFERENCE TO RELATED APPLICATIONS

This application claims the benefit of U.S. Provisional Patent Application Serial No. 63/509,192, filed June 20, 2023, the entire content of which is incorporated herein by reference.

### FIELD OF THE INVENTION

The present invention relates generally to the treatment of cardiac valve disease using prosthetic valves, and more particularly to a delivery system for a prosthetic valve device configured to replace a native heart valve.

### BACKGROUND

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based delivery systems. Such heart valve prostheses generally include a frame or stent and a prosthetic valve mounted within the frame. Such heart valve prostheses are delivered in a radially compressed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

In some circumstances, when releasing a heart valve prosthesis into a patient's vasculature, the energy dissipated can cause the valve to jump and can lead to movement and placement inaccuracy. Further, the heart valve prosthesis may experience backfolding or buckling during deployment.

Further, prosthetic valves (*e.g*., leaflets) of heart valve prostheses are generally made of organic tissue, *e.g.*, bovine or porcine tissue, and require onsite installation onto a delivery device. This is due to the need to store the heart valve prostheses in conditions specific to preserve the organic tissue. Typically, a detailed process must be performed in order to install a heart valve prosthesis on a delivery device at the geographic location of the procedure (*e.g.*, a hospital). The detailed process, however, can be cumbersome and costly due to the potential of damaging the heart valve prosthesis during the installation.

Thus, there is a need for improvements in delivery devices to minimize or prevent jumping, backfolding and/or buckling of the heart valve prosthesis during deployment that provides controlled release of both the inflow and the outflow end of the heart valve prosthesis. Further, there is a need for simplified devices and methods for loading a heart valve prosthesis onto a delivery device.

### BRIEF SUMMARY OF THE INVENTION

In accordance with an example hereof, a delivery system comprises an outer shaft including a capsule at a distal portion thereof, the capsule configured to restrain a prosthetic valve device therein, an inner shaft disposed within the outer shaft, and a first capture device disposed between the outer shaft and the inner shaft. The first capture device is configured to retain a portion of the prosthetic valve device. The first capture device comprises: a body including a central passage, a plurality of radial openings extending from the central passage radially outward to an outer surface of the body, and a plurality of slots in the outer surface of the body; and a plurality of capture loops, each capture loop extending radially outward through a corresponding radial opening, each capture loop configured to extend through a portion of the prosthetic valve device, and a distal end of each capture loop configured to be releasably restrained in a corresponding slot of the plurality of slots.

In another example hereof, the delivery system of any of the previous or subsequent examples herein further comprises a middle shaft, wherein the first capture device is coupled to the middle shaft.

In another example hereof, in the delivery system of any of the previous or subsequent examples herein, the first capture device further comprises a cover, the cover being rotatable between a first position in which the cover prevents the capture loops from escaping the slots and a second position in which the cover does not prevent at least some of the capture loops from escaping the slots.

In another example hereof, in the delivery system of any of the previous or subsequent examples herein, the cover includes at least one slot opening and at least one slot cover, wherein the cover is configured such that in the first position the at least one slot cover is aligned with a first slot of the plurality of slots and in the second position the at least one slot opening is aligned with the first slot of the plurality of slots.

In another example hereof, in the delivery system of any of the previous or subsequent examples herein, the body of the capture device includes a first quantity of slots, the cover includes a second quantity of slot openings, and the cover includes a third quantity of slot covers.

In another example hereof, in the delivery system of any of the previous or subsequent examples herein, the first quantity, the second quantity, and the third quantity are equal such that in the first position each of the slot covers is aligned with a corresponding slot of the plurality of slots and in the second position each of the slot openings is aligned with a corresponding slot of the plurality of slots.

In another example hereof, in the delivery system of any of the previous or subsequent examples herein, the second quantity is one and the third quantity is one such that in the first position the slot cover is aligned with all of slots of the plurality of slots and in the second position the slot opening is aligned with first one of the plurality of slots and the slot cover is aligned with all of the plurality of slots except the first one.

In another example hereof, in the delivery system of any of the previous or subsequent examples herein, the cover includes additional positions such that in each of the additional positions the slot opening is aligned with a different one of the plurality of slots.

In another example hereof, in the delivery system of any of the previous or subsequent examples herein, the second quantity is less than the first quantity such that in the first position the slot covers are aligned with all of the slots and in the second position the slot openings are aligned with a plurality of the slots that is fewer than the first quantity, wherein cover includes additional positions such that in each of the additional positions the slot openings are aligned with different slots of the plurality of slots.

In another example hereof, in the delivery system of any of the previous or subsequent examples herein, the first capture device further comprises: a cover lumen extending radially from the central passage, wherein the cover lumen intersects with each of the plurality of slots between a first end of the slot and a second end of the slot; a cover disposed within the cover lumen and being rotatable relative to the body within the cover lumen, the cover including a plurality of slot openings and a plurality of slot covers; a plurality of springs, each spring of the plurality of springs coupled to the first end of a corresponding slot of the plurality of slots; and a plurality of flaps, each flap disposed at the second end of a corresponding slot of the plurality of slots; wherein in a first position, each slot cover of the plurality of slot covers is aligned with corresponding slot of the plurality of slots, each spring is in a compressed state such that each spring is disposed between a corresponding slot cover and the first end of the corresponding slot, and the distal end of each capture loop is disposed in a corresponding slot between a corresponding slot cover and a corresponding flap at the second end of the corresponding slot; and wherein in second position, each slot opening of the plurality of slot openings is aligned with a slot of the plurality of slots such that each spring is released from its compressed state and decompresses, thereby pushing the distal end of the corresponding capture loop with sufficient force to push the capture loop through the corresponding flap of the plurality of flaps.

In another example hereof, the delivery system of any of the previous or subsequent examples further comprises a control shaft coupled to the cover, wherein the control shaft is configured to rotate the cover relative to the body of the capture device.

In another example hereof, in the delivery system of any of the previous or subsequent examples herein, the plurality of slots are L-shaped including a longitudinal portion and a circumferential portion, wherein the body is rotatable between a first position wherein the distal end of each capture loop is disposed in the circumferential portion of a corresponding slot and a second position wherein the distal end of each capture loop is aligned with the longitudinal portion of the corresponding slot.

In another example hereof, the delivery system of any of the previous or subsequent examples herein further comprises a pull wire coupled to proximal ends of the plurality of capture loops, wherein the pull wire is slidable within the outer shaft such that increased tension on the pull wire tightens the capture loops and decreased tension in the pull wire creates slack in the capture loops.

In another example hereof, the delivery system of any of the previous or subsequent examples herein further comprises a second capture device, wherein one of the first and second capture devices is a proximal capture device and the other of the first and second capture devices is a distal capture device, wherein the proximal capture device is configured to releasably retain a first end of the prosthetic valve device and the distal capture device is configured to releasably retain a second end of the prosthetic valve device.

In another example hereof, an implant assembly comprises an assembled configuration and an unassembled configuration. The implant assembly in the assembled configuration comprises a prosthetic valve device comprising a stent and a prosthetic valve coupled the stent, a first capture device coupled to a first end of the prosthetic valve device, and a second capture device coupled to a second end of the prosthetic valve device. The first capture device includes a capture body and a plurality of capture loops extending from the capture body through the first end of the prosthetic valve device and back to the capture body such that a distal end of each capture loop is releasably coupled to the capture body. The implant assembly in the assembled configuration is configured to be attached to a shaft of a delivery system. The prosthetic valve device is configured to be released from the first capture device and the second capture device after attachment to the shaft of the delivery system.

In another example hereof, in the implant assembly of any of the previous or subsequent examples herein, the first capture device further comprises a cover, the cover being rotatable between a first position in which the cover prevents the capture loops from escaping the slots and a second position in which the cover does not prevent at least some of the capture loops from escaping the slots.

In another example hereof, in the implant assembly of any of the previous or subsequent examples herein, the cover includes at least one slot opening and at least one slot cover, wherein the cover is configured such that in the first position the at least one slot cover is aligned with a first slot of the plurality of slots and in the second position the at least one slot opening is aligned with the first slot of the plurality of slots.

In another example hereof, in the implant assembly of any of the previous or subsequent examples herein, the body of the capture device includes a first quantity of slots, the cover includes a second quantity of slot openings, and the cover includes a third quantity of slot covers.

In another example hereof, in the implant assembly of any of the previous or subsequent examples herein, the first quantity, the second quantity, and the third quantity are equal such that in the first position each of the slot covers is aligned with a corresponding slot of the plurality of slots and in the second position each of the slot openings is aligned with a corresponding slot of the plurality of slots.

In another example hereof, in the implant assembly of any of the previous or subsequent examples herein, the second quantity is one and the third quantity is one such that in the first position the slot cover is aligned with all of slots of the plurality of slots and in the second position the slot opening is aligned with first one of the plurality of slots and the slot cover is aligned with all of the plurality of slots except the first one.

In another example hereof, in the implant assembly of any of the previous or subsequent examples herein, the cover includes additional positions such that in each of the additional positions the slot opening is aligned with a different one of the plurality of slots.

In another example hereof, in the implant assembly of any of the previous or subsequent examples herein, the second quantity is less than the first quantity such that in the first position the slot covers are aligned with all of the slots and in the second position the slot openings are aligned with a plurality of the slots that is fewer than the first quantity, wherein cover includes additional positions such that in each of the additional positions the slot openings are aligned with different slots of the plurality of slots.

In another example hereof, in the implant assembly of any of the previous or subsequent examples herein, the first capture device further comprises: a cover lumen extending radially from the central passage, wherein the cover lumen intersects with each of the plurality of slots between a first end of the slot and a second end of the slot; a cover disposed within the cover lumen and being rotatable relative to the body within the cover lumen, the cover including a plurality of slot openings and a plurality of slot covers; a plurality of springs, each spring of the plurality of springs coupled to the first end of a corresponding slot of the plurality of slots; and a plurality of flaps, each flap disposed at the second end of a corresponding slot of the plurality of slots; wherein in a first position, each slot cover of the plurality of slot covers is aligned with corresponding slot of the plurality of slots, each spring is in a compressed state such that each spring is disposed between a corresponding slot cover and the first end of the corresponding slot, and the distal end of each capture loop is disposed in a corresponding slot between a corresponding slot cover and a corresponding flap at the second end of the corresponding slot; and wherein in a second position, each slot opening of the plurality of slot openings is aligned with a slot of the plurality of slots such that each spring is released from its compressed state and decompresses, thereby pushing the distal end of the corresponding capture loop with sufficient force to push the capture loop through the corresponding flap of the plurality of flaps.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings may not be to scale.
FIG. 1 is a side view of an exemplary prosthetic valve device that may be utilized in embodiments hereof.
FIG. 1A is a perspective view of the prosthetic valve device of FIG. 1.
FIG. 1B is an end view of the prosthetic valve device of FIG. 1.
FIG. 2 is a side view of a delivery system according to an embodiment hereof.
FIG. 2A is a perspective view of the delivery system of FIG. 2.
FIG. 2B is a cross-sectional view of the delivery system of FIG. 2 taken along line B-B of FIG. 2.
FIG. 3A is a side perspective view of an implant assembly in a storage container.
FIG. 3B is a side perspective view of the implant assembly of FIG. 3A being loaded to the delivery system of FIG. 2.
FIG. 3C is a side perspective view of the implant assembly of FIG. 3A loaded onto the delivery system of FIG. 2.
FIG. 4 is a side perspective view of the delivery system of FIG. 2 and the prosthetic valve device of FIG. 1 prior to loading the prosthetic valve device on the delivery system.
FIG. 5 is a side perspective view of the delivery system of FIG. 2 and the prosthetic valve device of FIG. 1 with the distal capture device of the delivery system engaged or attached to a second end of the prosthetic valve device.
FIG. 6 is a side perspective view of the delivery system of FIG. 2 with the prosthetic heart device of FIG. 1 loaded onto the delivery system of FIG. 2.
FIG. 7 is a side perspective view of the delivery system of FIG. 2 and the prosthetic valve device of FIG. 1 as the prosthetic valve device is being collapsed and retracted into a loading funnel.
FIG. 8 is a side perspective view of the delivery system of FIG. 2 and the prosthetic valve device of FIG. 1 in which the prosthetic valve device is enclosed in a retractable capsule of the delivery system in a radially compressed configuration and is ready for intraluminal insertion or delivery to a desired anatomic site.
FIG. 9 is a side perspective view of the delivery system of FIG. 2 and the prosthetic valve device of FIG. 1 in which the retractable capsule is partially retracted, and the prosthetic valve device is partially expanded during deployment thereof, wherein the distal capture device of the delivery system is still engaged or attached to the second end of the prosthetic valve device.
FIG. 10 is a side perspective view of the delivery system of FIG. 2 and the prosthetic valve device of FIG. 1 in which the retractable capsule is partially retracted, and the prosthetic valve device is partially expanded during deployment thereof, wherein the second end of the prosthetic valve device is fully deployed and is no longer engaged or attached to the distal capture device of the delivery system.
FIG. 11 is a side perspective view of the delivery system of FIG. 2 and the prosthetic valve device of FIG. 1 in which the retractable capsule is retracted to expose an entire length of the prosthetic valve device and the prosthetic valve device is partially expanded during deployment thereof, wherein the second end of the prosthetic valve device is fully deployed and is no longer engaged or attached to the distal capture device of the delivery system and wherein the proximal capture device of the delivery system is still engaged or attached to the first end of the prosthetic valve device.
FIG. 12 is a side perspective view of the delivery system of FIG. 2 and the prosthetic valve device of FIG. 1 in which the prosthetic valve device is fully expanded or deployed and released from the delivery system, wherein the first and second ends of the prosthetic valve device are fully deployed and are no longer engaged or attached to the proximal and distal capture devices, respectively, of the delivery system.
FIG. 13 is schematic illustration of the prosthetic valve device of FIG. 1 deployed from the delivery system of FIG. 2, showing the proximal and distal capture devices.
FIG. 14 is an enlarged side perspective view of a capture device in a first position according to embodiments herein.
FIG. 15 is an enlarged side perspective view of the capture device of FIG. 14 in a second position according to embodiments herein.
FIG. 16A is an enlarged side perspective view of a capture device in a first position according to embodiments herein.
FIG. 16B is an enlarged side perspective view of the capture device of FIG. 16A in a second position according to embodiments herein.
FIG. 16C is an enlarged end view of a cover of the capture device of FIGS. 16A and 16B.
FIG. 16D is a schematic cross-sectional illustration of a portion of a delivery device showing the capture device of FIGS. 16A-16C with a control shaft coupled to a cover of the capture device.
FIG. 17A is an enlarged side perspective view of a capture device in a first position according to embodiments herein.
FIG. 17B is an enlarged side perspective view of the capture device of FIG. 17A in a second position.
FIG. 18A is an enlarged side perspective view of a capture device in a first position according to embodiments herein.
FIG. 18B is an enlarged side perspective view of the capture device of FIG. 18A in a second position.
FIG. 18C is schematic cross-sectional illustration of the body of the capture device of FIGS. 18A and 18B according to embodiments herein.
FIG. 18D is a schematic cross-sectional illustration of a portion of a delivery device showing the capture device of FIGS. 18A-18C with a control shaft coupled to a cover of the capture device.
FIG. 19 is an enlarged side perspective view of a capture device according to embodiments herein.
FIG. 20 is an enlarged side perspective view of a capture device according to embodiments herein.
FIG. 21 is a top plan view of an embodiment of a capture loop.
FIG. 22 is a side view the capture loop of FIG. 21.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a delivery device. The following detailed description is merely exemplary in nature and is not intended to limit the invention of the application and uses of the invention. Furthermore, there is no intention to be bound by any expressed or implied theory presented in the preceding field of the invention, background, summary or the following detailed description.

As used in this specification, the singular forms "a", "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number value.

The terms "proximal" and "distal" herein are used with reference to the clinician using the devices. Therefore, "proximal" and "proximally" mean in the direction toward the clinician, and "distal" and "distally" mean in the direction away from the clinician. In other words, "proximal" and "proximally" mean in the direction towards the handle of the delivery system and "distal" and "distally" mean in the direction towards the distal tip of the delivery system.

Further, numerical terms such as "first", "second", "third", etc. used herein are not meant to be limiting such that use of the term "second" when referring to a part in the specification does not mean that there necessarily is a "first" of part in order to fall within the scope of the invention. Instead, such numbers are merely describing that the particular embodiment being described has a "first" part and a "second" part. The invention is instead defined by the claims, in which one or more of the numbered parts may be claimed.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components.

The principles of the invention may be practiced in any instance in which it is desired to deliver a medical device intraluminally to a desired anatomic site. For the purpose of discussion, the invention will generally be described in the context in which the medical device being loaded onto a delivery system and delivered intraluminally is a prosthetic valve. In an embodiment, the prosthetic valve is an infundibular reducer device configured to be implanted in the right ventricular outflow tract or the infundibulum, and corresponding embodiments of the invention are particularly useful for delivering the infundibular reducer device to the right ventricular outflow tract. The prosthetic valve device may be used in anatomic locations other than the infundibulum, such as the right ventricular outflow tract and other locations in or near the heart. The purpose of such devices is to allow replacement or prosthetic valves, such as pericardial heart valves, for example, having a smaller diameter than the diameter of the implanted site (e.g., the right ventricular outflow tract) to be implanted. However, other uses of the claimed invention, such as to deliver different medical devices to different locations in the body, are contemplated and are not limited to those discussed in the application.

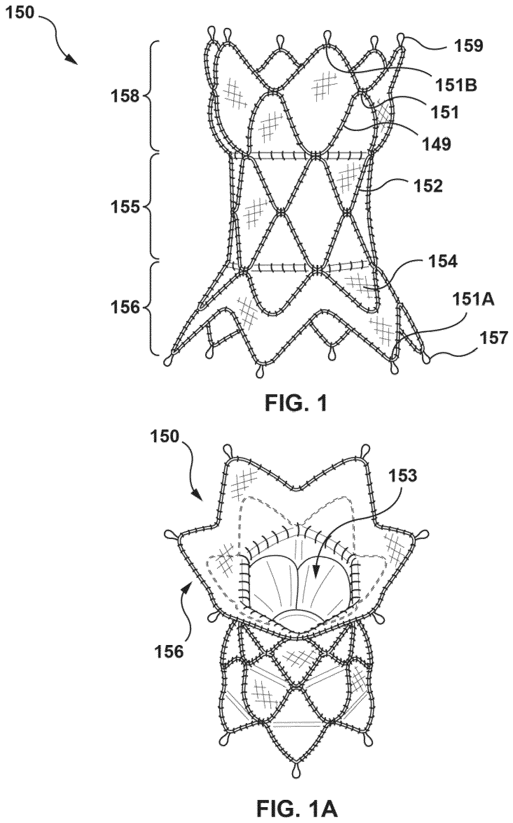

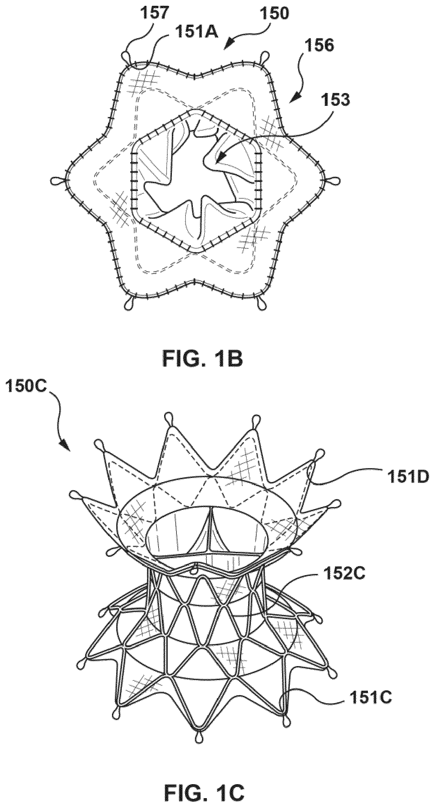

FIG. 1 is a side view of an exemplary prosthetic valve device 150 that may be deployed using embodiments of delivery systems described herein, while FIGS. 1A and 1B are perspective and end views, respectively, of the prosthetic valve device 150. The prosthetic valve device 150 is merely exemplary. It is understood that any number of alternate devices can be used with the delivery devices and methods described herein. For example, U.S. Patent No. 8,801,776 to House et al., which is incorporated by reference herein in its entirety, describes a device that may be delivered by delivery systems described herein. Similarly, the prosthetic valve devices described in U.S. Patent Application Publication No. 2021/0346158, which is incorporated by reference herein in its entirety, can be used with the delivery devices and methods described herein. In an embodiment, the prosthetic valve device 150 is the Medtronic HARMONY^{™} transcatheter pulmonary valve. In addition, the delivery systems described herein may also be used with other self-expanding prostheses such as other prosthetic heart valves, stent-graft prostheses, uncovered stents, bare metal stents, drug eluting stents, and any self-expanding structure.

As shown, prosthetic valve device 150 includes an expandable stent or frame 152 that supports a prosthetic valve component 153 within the interior of the stent 152. In embodiments hereof, the stent 152 is self-expanding or self-expandable to return to a radially expanded configuration from a radially compressed or constricted radially compressed configuration. The prosthetic valve device 150 is compressible to be mounted into a delivery system and expandable to fit a desired body lumen, such as the right ventricular outflow tract, for example. In the embodiment depicted in FIG. 1, the stent 152 has an expanded, longitudinally asymmetric hourglass configuration including three longitudinal sections or portions of a relatively enlarged first end 156, a relatively enlarged second end 158, and a midportion 155 extending between the first and second ends 156, 158. The first end 156 may also be referred to herein as the proximal or inflow end, and the second end 158 may also be referred to herein as the distal or outflow end. The midportion 155 is generally cylindrical in shape with a smaller diameter than the first and second ends 156, 158. In the example shown, the prosthetic valve component 153 is mounted within the midportion 155. Each longitudinal section or portion of stent 152 may be designed with a number of different configurations and sizes to meet the different requirements of the location in which it may be implanted. Each longitudinal section or portion of the stent 152 may have the same or different cross-portion which may be for example circular, ellipsoidal, rectangular, hexagonal, rectangular, square, or other polygonal shape, although at present it is believed that circular or ellipsoidal may be preferable when the prosthetic valve device is being provided for replacement of the aortic or mitral valve. As alternatives to the deployed asymmetric hourglass configuration of FIG. 1, the stent 152 may have a symmetric hourglass configuration, a generally tubular configuration, or other stent configuration or shape known in the art for valve replacement.

The example prosthetic device 150 also includes a plurality of first attachment members 157 on or near the first end 156 and a plurality of second attachment members 159 on or near the second end 158. More particularly, a first attachment member 157 is attached to each endmost crown 151A on or near the first end 156 and a second attachment member 159 is attached to each endmost crown 151B on or near the second end 158. Thus, the number of total attachment members are dependent upon the design and configuration of the prosthetic valve device 150, and thus may be higher or lower than shown in the embodiment of FIG. 1. Each attachment member of the first and second attachment members 157, 159 define an opening. Such attachment members may be loops formed from sutures, may be formed from the material used to form part of the stent 152, or other materials.

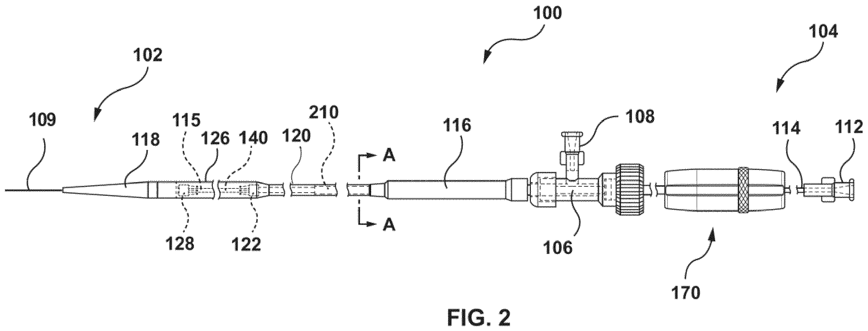

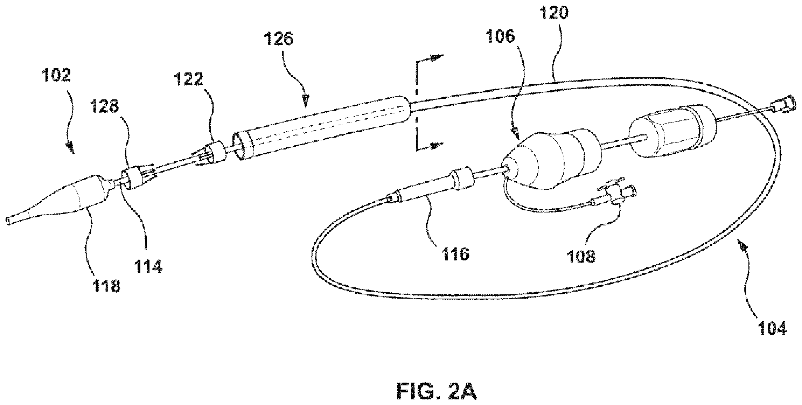

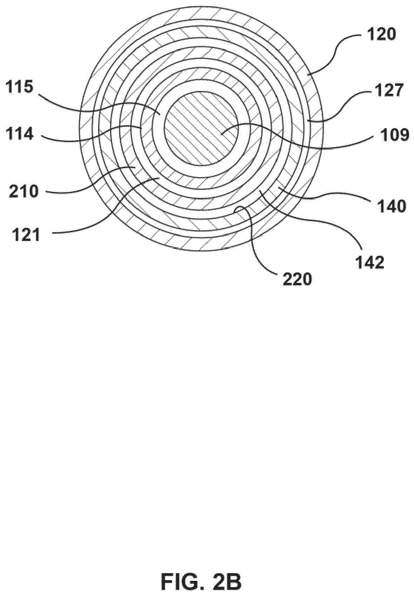

FIG. 2 is a side view of a delivery system 100 according to embodiments hereof. In general, the delivery system 100 is configured to receive and retain the prosthetic valve device 150 using a retention system (e.g., a proximal capture device 122 and/or a distal capture device 128, collectively referred to as capture devices 230) and percutaneously deliver the prosthetic valve device 150 in a compressed or radially compressed configuration to a treatment site *in situ.* More particularly, the delivery system 100 includes a proximal capture device 122 configured to engage or attach to the plurality of first attachment members 157 on or near the first end 156 of the prosthetic valve device 150 and a distal capture device 128 configured to engage or attach to the plurality of second attachment members 159 on or near the second end 158 of the prosthetic valve device 150. As described in more detail below, each capture device 230 generally includes a plurality of capture loops 225 configured for engagement with the prosthetic valve device 150 through respective attachment members 157 or 159. Each capture loop 225 is releasably retained within a respective slot 123 of the capture device 230 and is releasable from the respective slot 123 upon release of the prosthetic valve device 150.

FIG. 2 illustrates the delivery system 100 disposed over a guidewire 109 for illustrative purposes only. FIG. 2A is a schematic drawing of delivery system 100. With any of the systems and methods described herein, the guidewire 109 may be initially introduced into the target treatment site through a suitable body lumen to properly locate the desired position for the prosthetic valve device 150. The remainder of the delivery system 100 may then be guided along the guidewire 109 and into the site. The guidewire 109 may be, for example a 0.089 cm extra stiff guidewire as manufactured by Ampliated, Golden Valley, Minn., U.S.A.

The delivery system 100 includes a distal end generally designated by the reference numeral 102 and a proximal end generally designated by the reference number 104. The proximal end 104 of the delivery system 100 remains outside of the patient, and the distal end 102 is inserted into the patient and is delivered intravascularly to an area at or near a pulmonary valve inside the body. Other uses for the delivery system 100 in other areas of the body, however, are also contemplated. The proximal end 104 includes means for remotely controlling the distal end 102 of the delivery system 100, in particular relating to deploying or releasing the prosthetic valve device 150 from the delivery system 100 *in situ.*

The components of the proximal end 104 of the delivery system 100 may include those shown in FIGS. 2 and 2A, although additional and/or alternative components are also contemplated. The proximal end 104 may include a first rotating homeostasis valve 106, a side access port 108, an optional second rotating homeostasis valve (not shown), and a handle 170 including a guidewire lumen inlet 112. The first rotating homeostasis valve 106 grips or forms a fluid seal around an outer surface of the delivery system 100 to prevent blood or other fluid from leaking out of the delivery device at the proximal end or entry site into a patient and is configured to allow wires, devices and fluid to pass through. Furthermore, the first rotating homeostasis valve 106 may also control the components of the distal end as described in more detail below by rotation of a portion of the first rotating homeostasis valve 106. The side access port 108 is provided as a means for injecting contrast media or saline, for example, into the delivery system 100. The second rotating homeostasis valve also prevents blood or other fluid from leaking back through the delivery system 100 and is configured to allow wires, devices, and fluid to pass through. The handle 170 controls the components of the distal end 102 as described in more detail below by independent manipulation of two portions of the handle 170. Other alternative or additional components of the proximal end 104 of the delivery system 100 are also contemplated by the claimed invention.

FIG. 2B shows a cross-sectional view of the delivery system 100 of FIG. 2 taken along line A-A of FIG. 2. In an embodiment, the delivery system 100 includes at least three concentric tubular components, while the embodiment shown in FIG. 2B includes four concentric tubular components. More particularly, the delivery system 100 includes an inner shaft 114 which defines a guidewire lumen 115, a control shaft 210 which defines a control shaft lumen 121 and is slidingly disposed over the inner shaft 114, a middle shaft 140 which defines a middle shaft lumen 142 and is slidingly disposed over the control shaft 210, and an outer shaft 120 which includes an outer sheath or capsule 126 and defines a lumen 127 and is slidingly disposed over the middle shaft 140. The outer shaft 120 or the capsule 126 coupled to a distal end of the outer shaft 120 is configured to hold the prosthetic valve device 150 in a radially collapsed or compressed configuration during delivery. According to certain embodiments disclosed herein, the middle shaft 140 may operate as a control shaft, thereby reducing the number of tubular components to three (*e.g*., the inner shaft 114, the middle shaft 140 and the outer shaft 120) instead of the four tubular components (*i.e.*, the inner shaft 114, the control shaft 210, the middle shaft 140 and the outer shaft 120) illustrated in FIG. 2B. The delivery system 100 may further include at least one pull wire 220, described in further detail below. The delivery system 100 may further include an outer sleeve 116 that is disposed over a proximal portion of the outer shaft 120 to keep blood from leaking back around the delivery system 100.

The inner shaft 114 may include the tapered distal tip 118 attached thereto at its distal end and may be attached to the guidewire lumen inlet 112 at its proximal end such that the inner shaft 114 may be tracked over the guidewire 109. The inner shaft 114 may be independently advanced or retracted through other components of the delivery system 100 by moving the guidewire lumen inlet 112 to which it is attached. The tapered distal tip 118 serves to ease the passage of the delivery system 100 through the vasculature.

The middle shaft 140 is slidingly disposed over the inner shaft 114 and may be independently retractable with respect to the other shaft components of the delivery system 100. In this embodiment, the distal capture device 128 is coupled to a distal end of the middle shaft 140. In embodiments, both the distal capture device 128 and proximal capture device 122 are coupled to middle shaft 140, where the distal capture device 128 is coupled to the distal end of the middle shaft 140 and proximal capture device 122 is coupled to the middle shaft 140 proximal to where the distal capture device 128 is coupled to the middle shaft 140.

In the embodiment shown, the outer shaft 120 includes the retractable sheath or capsule 126 coupled to the distal end of the outer shaft 120. The outer shaft 120 is retractable via a retraction mechanism such as a knob of the first rotating homeostasis valve 106, thereby also retracting the capsule 126. Various other retraction mechanisms may be used, such as an axially slidable lever, a rotatable rack and pinion gear, or other known mechanisms. In this way, the outer shaft 120 and capsule 126 are retractable relative to the inner shaft 114 and the middle shaft 140 during deployment of the prosthetic valve device 150.

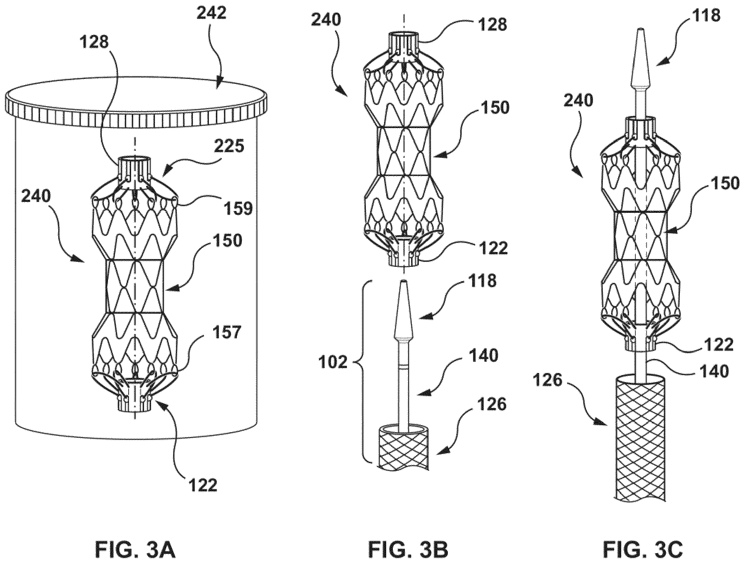

As discussed above, loading the prosthetic valve device 150 into the delivery system 100 at the geographic location of the prosthetic valve device implantation (*e.g.*, hospital) can be difficult and potentially cause damage to the prosthetic valve device 150. However, it is also difficult to pre-load the prosthetic valve device 150 into the delivery system 100 (*i.e.,* at the manufacturing site) due to the need to preserve the organic tissue of the prosthetic valve device 150. Therefore, in an embodiment hereof, the proximal and distal capture devices 122, 128 are pre-loaded onto or pre-connected to the prosthetic valve device 150 forming an implant assembly 240. The pre-connection of the proximal and distal capture devices 122, 128 to the prosthetic valve device 150 may occur at the manufacturer's site, thereby ensuring that the prosthetic valve device 150 is properly connected to the proximal and distal capture devices 122, 128. In other embodiments, the pre-connection of the proximal and distal capture devices 122, 128 to the prosthetic valve device 150 may occur at the geographic location of the prosthetic valve device implantation (*e.g*., hospital). As shown in FIG. 3A, the implant assembly 240 (*i.e.,* the prosthetic valve device 150 and the proximal and distal capture devices 122, 128) may be stored in a storage container 242, such as ajar, for shipment to the geographic location of the implantation procedure. The storage container 242 includes a preserving fluid. The preserving fluid can be any type of fluid that maintains the integrity and quality of the implant assembly 240. For example, if the prosthetic valve device 150 includes organic material, the preserving fluid may include glutaraldehyde, formaldehyde, or other suitable preserving fluids to maintain the integrity of the organic material.

As shown in FIGS. 3B and 3C, when ready for the implantation procedure, the implant assembly 240 may be removed from the storage container 242 at the geographic location of the implantation procedure, as shown in FIG. 3B. The proximal capture device 122 is aligned with the distal end 102 of the delivery system 100. The implant assembly 240 and the delivery system 100 are then moved relative to one another such that the implant assembly 240 is loaded over the inner shafts of the delivery system 100 (*e.g.*, the inner shaft 114 and the middle shaft 140), as shown in FIG. 3B. The implant assembly 240 is then connected to the delivery system 100, as shown in FIG. 3C. In an example, the implant assembly 240 may be connected to the delivery system 100 via a snap fit connection between the proximal capture device 122 and/or the distal capture device 128 and the shaft over which the proximal and distal capture devices 122, 128 are disposed, such as the middle shaft 140. For example, and not by way of limitation, in an embodiment with the proximal and distal cartridges 122, 128 coupled to the middle shaft 140, the middle shaft 140 may include one or more bumps or protrusions extending outwardly from an outer surface thereof. The proximal capture device 122 and/or the distal capture device 128 may include a corresponding one or more indentations. When the implant assembly 240 is moved proximally relative to the delivery system 100, the proximal capture device 122 and/or the distal capture device 128 may be forced over the one or more protrusions until the one or more protrusions extend into the corresponding one or more indentations, thereby attaching the implant assembly 240 to the middle shaft 140. The implant assembly 240 is then ready for further loading and delivery, as explained below.

It should be understood that other ways to attach the implant assembly 240 to the delivery system 100 are also contemplated. For example, and not by way of limitation, it is contemplated that the proximal and distal capture device 122, 128 may be pre-attached to the prosthetic valve 150 and to a portion of the middle shaft 140 such that the implant assembly 240 includes the proximal and distal capture device 122, 128, the prosthetic valve device 150, and the portion of the middle shaft 140. The implant assembly 240 may then be attached to the delivery system 100 by attaching the portion of the middle shaft 140 to the remainder of the middle shaft 140. The connection may be a snap-fit connection, a threaded connection, a hinged connection, or other suitable connections known to those skilled in the art.

Further, the distal tip 118 may be initially unattached to the inner shaft 114 such that when inserting the inner shaft 114/middle shaft 140 through the implant assembly 240, the distal tip 118 is not attached to the inner shaft 114. This enables the proximal and distal capture device 122, 128 to slide over the inner shaft 114, whereas sliding the proximal and distal capture device 122, 128 over the distal tip 118 may be difficult due to the size of the distal tip 118. The distal tip 118 can be attached to the inner shaft 114 after the implant assembly 240 has been attached to the inner shaft 114/middle shaft 140. The distal tip 118 may be attached to the inner shaft 114 via a snap-fit connection, a threaded connection, or other suitable connections known to those skilled in the art. In other embodiments the distal tip 118 may be integrated into the distal capture device 128 of the implant assembly 240 such that when the implant assembly 240 is coupled to the delivery system 100, the distal tip 118 is also coupled to the delivery system 100.

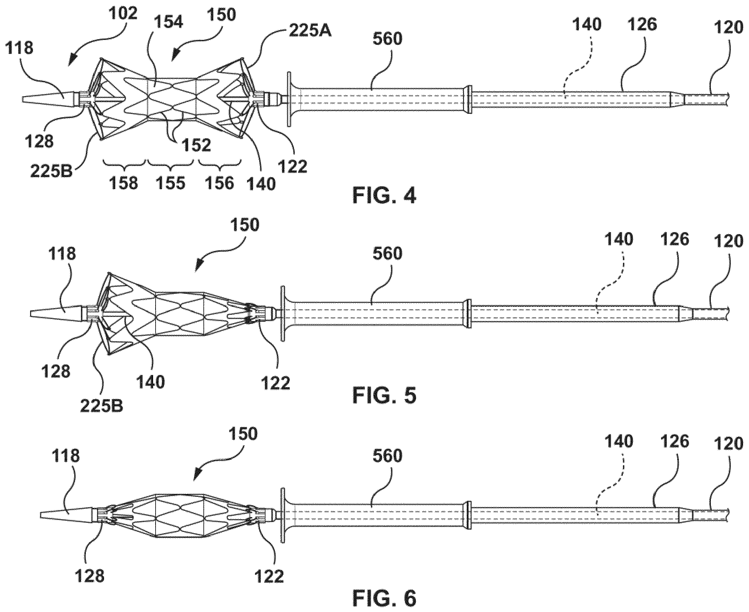

In other embodiments, the prosthetic valve device 150 may be coupled to the delivery system 100 at the geographic location of the implantation procedure. In such embodiments, distal tip 118, the inner shaft 114, and the middle shaft 140 are inserted through the prosthetic valve device 150 such that the prosthetic valve device 150 is disposed over the inner and middle shafts 114, 140 and between the proximal capture device 122 and the distal capture device 128, as shown in FIG. 4. The proximal capture device 122 is coupled to the first end 156 of the prosthetic valve device 150 by looping a plurality of capture loops 225A through corresponding openings in the plurality of first attachment members 157, and the distal capture device 128 is coupled to the second end 158 of the prosthetic valve device 150 by looping, or otherwise engaging, a plurality of capture loops 225B through corresponding openings in the plurality of second attachment members 159, as shown in FIG. 4. The first and second ends 156, 158 of the prosthetic valve device 150 may then be radially compressed, as shown in FIGS. 5 and 6. In embodiments described herein, the first and second ends of the prosthetic valve device 150 may be radially compressed by applying tension to the capture loops 225A, 225B, such as by use of a pull wire 220, described below. In embodiments herein, the pull wire 220 extends proximally to the handle 170, where tension on the pull wire 220, and hence the corresponding capture loops 225A or 225B, may be increased or decreased. Although the method of loading described herein includes attaching the first end 156 of the prosthetic valve device 150 to the delivery system 100 prior to attaching the second end 158 of the prosthetic valve device 150 to the delivery system, the order may be reversed and the second end 158 may be attached prior to the first end 156.

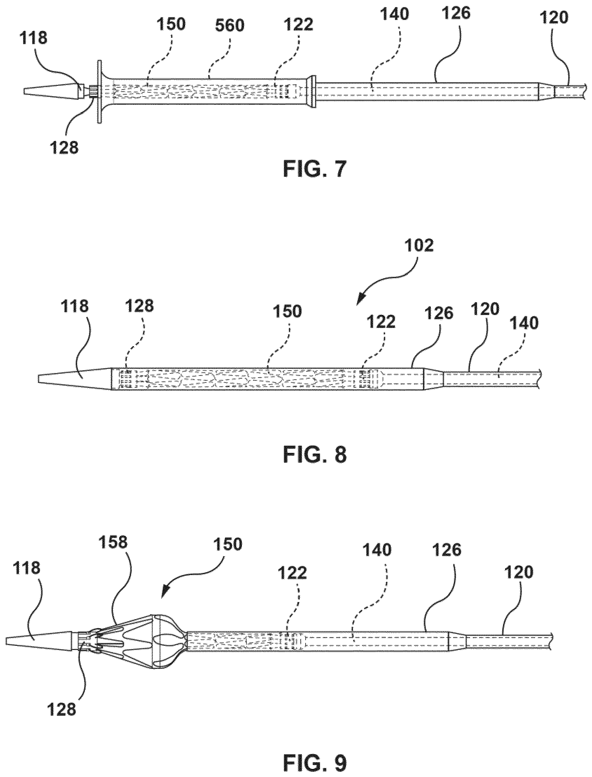

After both the first and second ends 156, 158 of the prosthetic valve device 150 are attached to the delivery system 100, a loading funnel 560 is used to further compress the prosthetic valve device 150 for eventual enclosure in the delivery system 100. Prior to loading the prosthetic valve device 150 onto delivery system 100, the loading funnel 560 is disposed over the capsule 126 proximal of the proximal capture device 122. The loading funnel 560 may be disposed over the capsule 150 by sliding the loading funnel 560 proximally over the distal tip 118 and the capsule 126. In other embodiments, the loading funnel 560 may include two parts that are coupled together over the capsule 126. After both the first and second ends 156, 158 of the prosthetic valve device 150 are coupled to delivery system 100, the loading funnel 560 may be advanced distally causing the prosthetic valve device 150 to be compressed by advancement of the loading funnel 560, as shown in FIG. 7. Alternatively, the assembly of the middle shaft 120, the inner shaft 114, and the prosthetic valve device 150 may be retracted proximally into the loading funnel 560. FIG. 7 is a side perspective view of the delivery system 100 with the prosthetic valve device 150 completely collapsed or enclosed within the loading funnel 560.

The assembly of the middle shaft 140, the inner shaft 114, and the prosthetic valve device 150 may be further retracted proximally until the collapsed prosthetic valve device 150 is disposed within the capsule 126 and the distal tip 118 fits into the distal end of the capsule 126, as shown in FIG. 8. FIG. 8 shows the distal end 102 of the delivery system 100 as it can be inserted intraluminally to a desired anatomic site for delivery of the prosthetic valve device 150. At this point, the capsule 126 contains the collapsed prosthetic valve device 150 in order to compress the prosthetic valve device 150 to have a profile reduced enough to be inserted into a desired anatomic site and to provide a smooth profile for insertion. The loading funnel 560 has been removed from the delivery system 100 by distally advancing the loading funnel 560 until it is removed from the delivery system 100.

In order to deploy or release the prosthetic valve device 150 once it has been positioned at its desired location *in situ* in the vasculature or in a heart valve annulus, for example, the capsule 126 is either retracted proximally, or the assembly of the middle shaft 120, the inner shaft 114, and the prosthetic valve device 150 is pushed out the distal end of the capsule 126. Notably, the pushability of the assembly of the outer shaft 120, the inner shaft 114, and the prosthetic valve device 150 is improved with both the first and second ends 156, 158 of the prosthetic valve device 150 being constrained by the proximal and distal capture devices 122, 128 because such constraint prevents any inadvertent buckling of the prosthetic valve device 150 that may otherwise occur when being pushed in a distal direction without both the first and second ends of the prosthetic valve device 150 being constrained by the proximal and distal capture devices 122, 128. FIG. 9 shows deployment of the prosthetic valve device 150 is initiated via retraction of the capsule 126. FIG. 9 is a side perspective view of the delivery system 100 in which the capsule 126 is partially retracted to expose at least the second end 158 of the prosthetic valve device 150 and the exposed length of the prosthetic valve device 150 is partially expanded. The second end 158 of the prosthetic valve device 150 is still engaged or attached to the distal capture device 128 of the delivery system 100. Although the capsule 126 is shown as only partially retracted, an operator may retract the capsule 126 to expose the entire length of the prosthetic valve device 150. At this stage of deployment, positioning of the delivery system 100 may still be adjusted and/or the capsule 126 may be distally advanced to recapture the prosthetic valve device 150. More particularly, even if the capsule 126 is retracted to expose the entire length of the prosthetic valve device 150, the second end 158 of prosthetic valve device 150 may essentially be controlled via the distal capture device 128. In other words, tension on the capture loops 225B may be controlled, such as by the pull wire 220, such that the profile thereof of the prosthetic valve device may be sufficiently reduced to enable the capsule 126 to be distally advanced to recapture the prosthetic valve device 150 and thus allow repositioning thereof *in situ.*

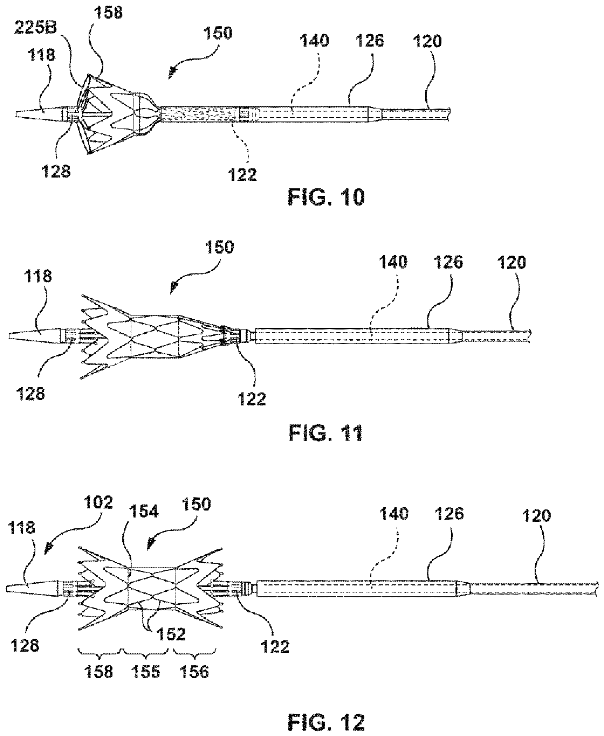

FIG. 10 shows the second end 158 of the prosthetic valve device 150 with tension on the capture loops 225B loosened such that the second end 158 is permitted to radially expand. However, the capture loops 225B have not been released from the prosthetic valve device 150. Thus, tension could be re-applied to radially compress the second end 158 of the prosthetic valve device 150 for recapture and re-positioning thereof, if desired.

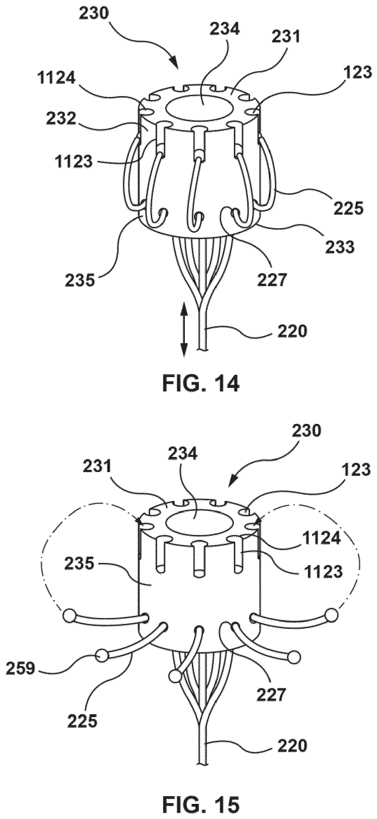

Once the prosthetic heart valve device 150 is properly positioned, the second end 158 of the prosthetic valve device 150 is released from the distal capture device 128 and permitted to self-expand, as shown in FIG. 11. More particularly, the distal capture loops 225B are released from slots 123 in the distal capture device 128 (see FIGS 14-15), and the second attachment members 159 of the prosthetic valve device 150, thereby releasing the second end 158 of the prosthetic valve device 150 from the delivery system 100. In FIG. 11, the capsule 126 is fully retracted to expose an entire length of the prosthetic valve device 150, with the first end 156 of the prosthetic valve device 150 still engaged or attached to the proximal capture device 122 of the delivery system 100. However, as stated above, the capsule 126 may have been fully retracted to expose an entire length of the prosthetic valve device 150 prior to releasing the second end 158 of the prosthetic valve device 150 from the proximal capture device 128.

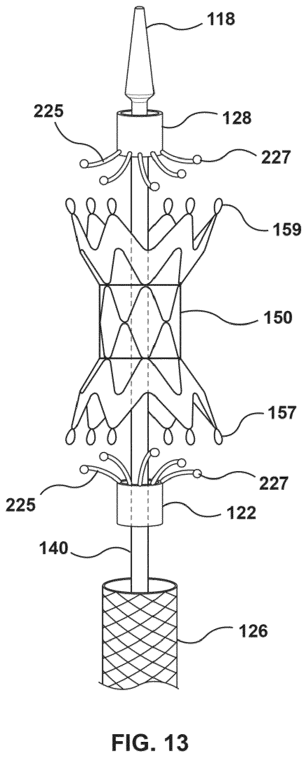

As shown in FIG. 12, the first end 156 of the prosthetic valve device 150 is then released from the proximal capture device 122 and permitted to self-expand. FIG. 13 shows a close-up view of the distal end 102 of the delivery system 100 with the prosthetic valve device released therefrom.

Various devices and methods may be used to release the proximal and distal capture loops 225A, 225B from the slots 123 as described in more detail below. In particular embodiments, the control shaft 210 may control portions of the capture devices 230 to release the capture loops 225 from the slots 123. In other embodiments, the middle shaft 140 may control portions of the capture devices 230 to release the capture loops 225 from the slots 123. In other embodiments, expansion of the prosthetic valve device 150 releases the capture loops 225 from the slots 123 of the capture devices 230. In other embodiments, the capture loops 225 may comprise shape memory material such that the capture loops 225 release themselves from the slots 123 upon release of a force holding the capture loops in the slots 123.

FIGS. 14-20 illustrate various embodiments of the capture device 230. Features of the various embodiments of the capture devices 230 described may be interchanged, added, or removed where appropriate. Features not described with respect to a particular embodiment may be included from another embodiment. The capture devices 230 described with respect to FIGS. 14-20 may be used as the proximal capture device 122 and/or the distal capture device 128 of the delivery system 100. Further, the proximal capture device 122 may be one embodiment of a capture device 230 and the distal capture device 128 may be a different embodiment of a capture device 230.

FIGS. 14 and 15 show an embodiment of a capture device 230. The capture device 230 shown in FIGS. 14 and 15 can be used as the proximal capture device 122 and/or the distal capture device 128. FIG. 14 shows the capture device 230 in a first or engaged position, and FIG. 15 shows the capture device 230 in a second or disengaged position. The capture device 230 includes a includes a body 232, a central passage 234, plurality of slots 123, and a plurality of capture loops 225, where each capture loop 225 is releasably retained within a corresponding slot 123 of the capture device 230. The body 232 includes a first end surface 231, a second end surface 233 opposite the first end surface 231, and an outer surface 235 between the first end surface 231 and the second end surface 233. In the embodiments shown, the body 232 is generally cylindrically shaped, but that is not meant to be limiting. The capture device 230 further includes a plurality of openings 227 extending from the central passage 234 to an outer surface 235 of the body 232. As shown in FIGS. 14 and 15, the capture loops 225 may be connected to each other proximal of the capture device 230 to a pull wire 220, which runs proximally to the handle 170 of the delivery system 100, either through the control shaft lumen 121 or the middle shaft lumen 142, or other appropriate lumen. From the pull wire 220, the capture loops 225 extend distally into the central passage 234 of the body 232, out of corresponding openings 227, and loop through a portion of the prosthetic valve device (e.g., the first attachment members 157 or the second attachment members 159) and into the corresponding slots 123. In the embodiment shown, the slots 123 include a slot opening 1124 at the first end 231 of the body 232. Further, slits 1123 in the outer surface 235 of the body 232 open into the slots 123. The slot openings 1124 are sized such that enlarged ends 259 of the capture loops 225 may enter and exit the slots 123 via the slot openings 1124. The slits 1123 are sized such that the enlarged ends 259 cannot enter or exit the slots 123 via the slits 1123.

According to embodiments herein, increased tension on the pull wire 220 tightens the capture loops 225 and decreased tension on the pull wire 220 loosens the capture loops 225. Moreover, according to embodiments herein, tension on the pull wire 220 is increased during loading to tighten capture loops 225 to reduce the diameter of the prosthetic valve device 150 where the capture loops 225 are coupled thereto. Further, tension on the pull wire 220 is decreased during implant deployment to create slack in the capture loops 225 and gradually allow the crowns of the prosthetic valve device 150 to expand, until the prosthetic valve device 150 is fully expanded. The capture loops 225 may be released from the slots 123 via expansion of the prosthetic valve device 150, via shape memory, and/or via articulation of the control shaft 210 or the middle shaft 140, as explained in various embodiment below. According to various embodiments disclosed herein, capture loops 225 may be polymeric (e.g., sutures), metallic, or other suitable materials.

According to various embodiments disclosed herein, at least one of the proximal capture device 122 and the distal capture device 128 have adjustable-length capture loops 225. The capture loops 225 are adjustable via the pull wire 220, as shown in IFGS. 14 and 15. In other embodiments, the capture loops 225 are fixed length such that a first end of each capture loop 225 may be attached to the body 232 and the second end of each capture loop 225 is inserted into the corresponding slot 123. In embodiments, both the proximal capture device 122 and the distal capture device 128 have adjustable-length capture loops 225. In other embodiments, only the distal capture device 128 includes adjustable-length capture loops 225, and the proximal capture device 122 includes fixed-length capture loops 225.

As explained above, FIG. 14 shows the capture device 230 in a first or engaged position, and FIG. 15 shows the capture device 230 in a second or disengaged position. In the first position, the capture loops 225 exit the openings 227 radially outwardly, loop through a portion of the prosthetic valve device (e.g., first or second attachment members 157, 159, not shown in FIGS. 14 and 15), and back into the slots 123. In the second position, the ends 259 of the capture loops 225 are released from the slots 123, such that the prosthetic heart valve device 150 is released from the delivery system 100. In the embodiment of FIGS. 14 and 15, the capture loops are released from the slots 123 either by expansion of the prosthetic valve device 150 pulling the capture loops 225 out of the slots 123, or by making the capture loops 225 from a shape memory material and pre-setting the shape of the capture loops 225 to the second or disengaged position of FIG. 15. Thus, when tension on the pull wire 220 is released and/or the capture device 225 is not covered by the capsule 126, the capture loops 225 return to the second position of FIG. 15, thereby releasing the prosthetic valve device 150.

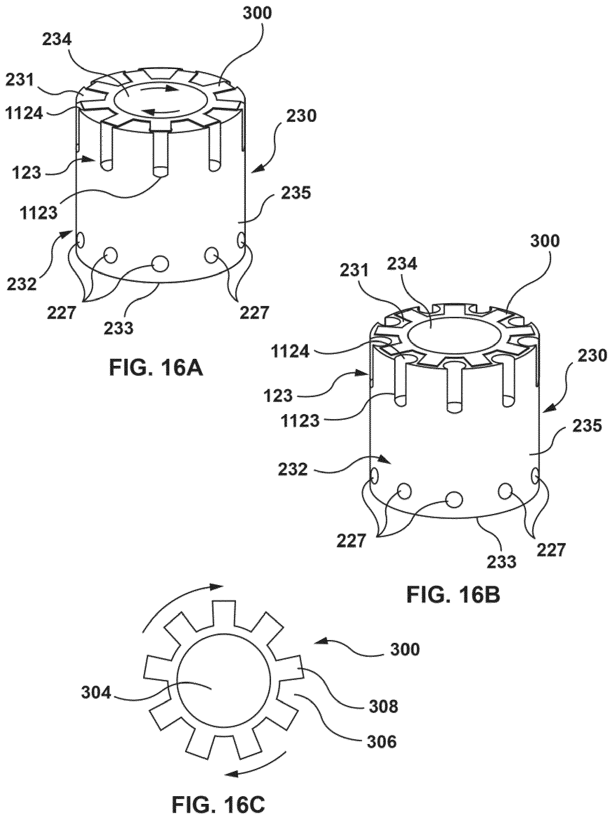

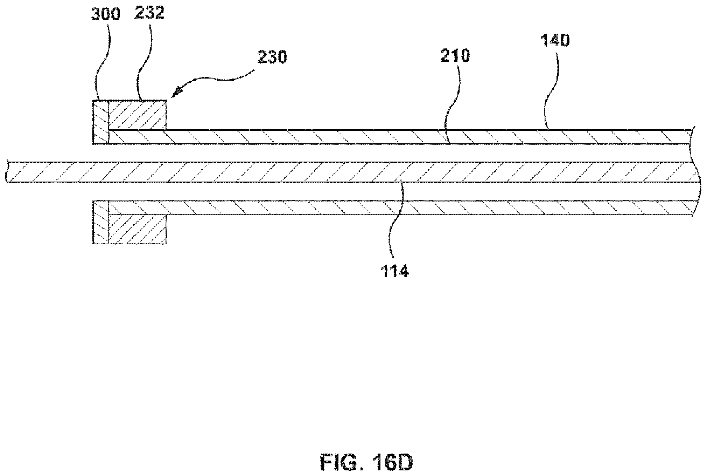

FIGS. 16A-16C show a capture device 230 according to another embodiment herein. The capture loops 225 are not shown in FIGS 16A-16C for clarity but may be the capture loops 225 described with respect to FIGS. 14 and 15. The capture device 230 of FIGS. 16A-16C includes the body 232, the central passage 234, the slots 123, and the openings 227 as described above. Further, the capture device 230 of FIGS. 16A-16C includes a cover 300 disposed at or coupled to the first longitudinal end surface 231 of the body 232. The cover 300 is shown in greater detail in FIG. 16C and includes a body 302, a central longitudinal opening 304 extending through the center of the body 302, a plurality of slot openings 306 extending through the body 302 and evenly distributed radially around central longitudinal opening 304. The plurality of slot openings 306 further define edges of a plurality of slot covers 308 evenly radiating outward from central longitudinal opening 304 and formed from the portion of the body 302 occupying the space between each slot opening 306. As illustrated in FIGS. 16A and 16B, the body 302 is disposed at the first longitudinal end surface 231 of the body 232 and centered on the body 232 such that the central longitudinal opening 304 is aligned with the central passage 234 of the body 232. The cover 300 is rotatable relative to the body 302. As shown in FIG. 16D, in embodiments, the cover 300 may be operatively coupled to the control shaft 210 such that rotation of the control shaft 210 (e.g., by rotating a handle actuator) rotates the body 302 of the cover 300 relative to the body 232.

The cover 300 is configured to be rotated to two distinct positions relative to the body 232 of the capture device 230. FIG. 16A illustrates the cover 300 in a first position, and FIG. 16B illustrates the cover 300 in a second position. In the first position, according to the embodiment illustrated in FIG. 16A, each slot cover 308 covers a corresponding slot opening 1124 of the body 232. Thus, in the first position, the slot covers 308 prevent the enlarged ends 259 of the capture loops 225 from exiting the slots 123 through the slot openings 1124. In the second position, according to the embodiment illustrated in FIG. 16B, each slot opening 306 aligns with a corresponding slot opening 1124 of the capture device 230. Thus, in the second position, with the slot openings 1124 uncovered, the ends 259 of the capture loops 225 may escape the slots 123 through the slot openings 1124 of the capture device 230 and the slot openings 306 of the cover 300.

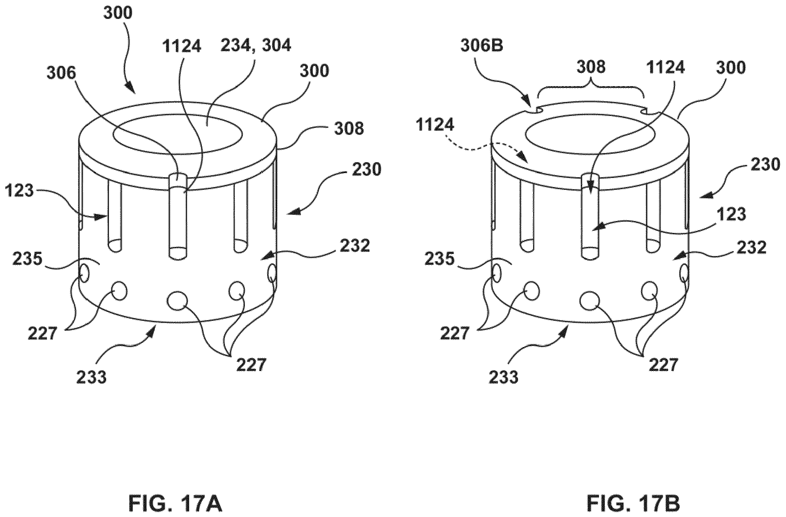

FIG. 17A shows a capture device 230 according to another embodiment herein. The capture loops 225 are not shown in FIG. 17A for clarity but may be the capture loops 225 described with respect to FIGS. 14 and 15. The capture device 230 of FIG. 17A is similar to the capture device 230 of FIG. 16A such that like features will not be described again. Generally, the capture device 230 of FIG. 17A includes the body 232, the central passage 234, the slots 123, and the openings 227 as described above. Further, the capture device 230 of FIG. 17A includes the cover 300 disposed at or coupled to the first longitudinal end surface 231 of the body 232. However, in the embodiment of FIG. 17A, the cover 300 includes a single slot opening 306. Thus, in the embodiment of FIG. 17A, the cover 300 is rotatable relative to the body 232 to multiple distinct positions, such as by operation of the control shaft 210 as shown in FIG. 16D, such that the single slot opening 306 is aligned with only a single one of the plurality of slot openings 1124 in the body 232 in each position. Thus, to couple the prosthetic valve device 150 to the capture device 230 and release the prosthetic valve device 150 from the capture device 230, the cover 300 is rotated such that the slot opening 306 is aligned with one of the slot openings 1124 one slot opening 1124 at a time. This enables simplified coupling of the prosthetic valve device 150 to the capture device 230 because a first capture loop 225 may be looped through the prosthetic valve device 150 (such as through one of the attachment loops 157, 159), the enlarged end 259 of the capture loop 225 slid through the slot opening 306 and a first slot opening 1124, and into a first slot 123. The cover 300 is rotated to such that one slot opening 306 of the cover is aligned with a second slot opening 1124. Thus, the slot cover 308 of the cover 300 is aligned with the other slot openings 1124 other than the second slot opening 1124. Thus, the end 259 of the capture loop 225 cannot escape the first slot opening 1124. This enables the capture loops 225 to be disposed within the slots 123 one at a time without risk of the capture loops 225 escaping the slots 123. This is opposed to the embodiment of FIGS. 16A-16C in which all of the capture loops 225 must be disposed within the slots 123 prior to rotating the cover 300 to cover the slot openings 1124. The single slot opening 306 of the cover 300 also enables a more controlled released of the prosthetic valve device 150 from the capture device 230 as the capture loops 225 are released one at a time.

FIG. 17B shows a capture device 230 according to another embodiment herein. The capture loops 225 are not shown in FIG. 17B for clarity but may be the capture loops 225 described with respect to FIGS. 14 and 15. The capture device 230 of FIG. 17B is similar to the capture device 230 of FIGS. 16A-16C and FIG. 17A such that like features will not be described above. Generally, the capture device 230 of FIG. 17B includes the body 232, the central passage 234, the slots 123, and the openings 227 as described above. Further, the capture device 230 of FIG. 17B includes the cover 300 disposed at or coupled to the first longitudinal end surface 231 of the body 232. However, in the embodiment of FIG. 17B, the cover 300 includes three slot openings 306. Thus, in the embodiment of FIG. 17B, the cover 300 is rotatable relative to the body 232 to multiple distinct positions, such as by operation of the control shaft 210 as shown in FIG. 16D, such that the three slot openings 306 are aligned with three of the plurality of slot openings 1124 in the body 232 in each position. Thus, to couple the prosthetic valve device 150 to the capture device 230 and release the prosthetic valve device 150 from the capture device 230, the cover 300 is rotated such that the slot openings 306 are aligned with a corresponding three of the slot openings 1124 at a time. Similar to the embodiment of FIG. 17A, this enables simplified coupling of the prosthetic valve device 150 to the capture device 230 because three capture loops 225 may be looped through the prosthetic valve device 150 (such as through corresponding attachment loops 157, 159), the enlarged ends 259 of the capture loops 225 slid through the three slot openings 306 and a corresponding three slot openings 1124, and into a corresponding three slots 123. The cover 300 is rotated to such that three slot openings 306 of the cover 300 are aligned with a different set of three slot openings 1124 of the body 302. Thus, the slot covers 308 of the cover 300 are aligned with the other slot openings 1124 other than second set of three slot openings 1124. Thus, the ends 259 of the capture loops 225 cannot escape the first set of three slot openings 1124. This enables the capture loops 225 to be disposed within the slots 123 three at a time without risk of the capture loops 225 escaping the slots 123. The three slot openings 306 of the cover 300 also enable a more controlled released of the prosthetic valve device 150 from the capture device 230 as the capture loops 225 are released three at a time.

It should be understood that while FIGS. 17A and 17B show a single slot opening 306 and three slot openings 306, respectively, this is not meant to be limiting, and a different number of slot openings 306 may be utilized. For example, and not by way of limitation, in other embodiments, the cover may include slot openings 306 equal to half of the slot openings 1124 in the body 232, or equal to one-third of the slot openings 1124, or one-quarter of the slot openings 1124, or other suitable amounts. Further, the slot openings 306 need not be evening distributed around the body 302 of the cover 300. For example, and not by way of limitation, in other embodiments, the three slot openings 306 in the embodiment of FIG. 17B may be adjacent to each other such that the three slot openings 306 align with three consecutive slots 1124. Such an arrangement (or similar arrangements with different quantities of slot openings 306) may enable loading and release of certain portions of the prosthetic valve device 150 prior to loading and release of other portions of the prosthetic valve device 150.

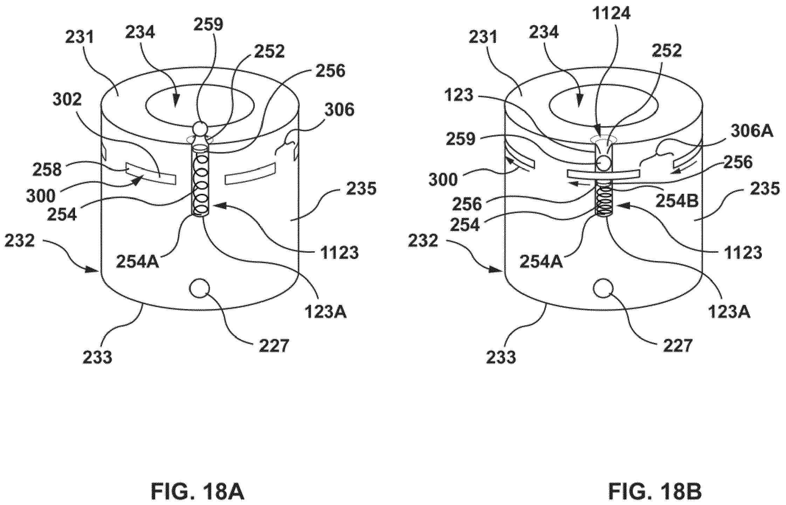

FIGS. 18A-18D show another embodiment of a capture device 230. The capture loops 225 are not shown in FIGS 18A-18D for clarity but may be the capture loops 225 described with respect to FIGS. 14 and 15. The capture device 230 of FIGS. 18A-18D includes the body 232, the central passage 234, the slots 123, the openings 227, the plurality of slot openings 1124, and the plurality of slits 1123, as described above. Further, in the embodiment of FIGS. 18A-18D, each slot opening 1124 includes a flexible flap 252 that provides access to the corresponding slot 123. The embodiment of FIGS. 18A-18D further includes a plurality of springs 254, each disposed within a corresponding slot 123. Each spring 254 is coupled to an end portion 123A of the slot 123 at a first end 254A of spring 254. A platform or pusher 256 is coupled to a second end 254B of the spring 254. The pusher 256 is suitably sized to freely traverse throughout the length of slot 123 and suitably sized to force the releasable end 259 of each corresponding capture loop 225 out of the slot 123 by decompression of the spring 254.

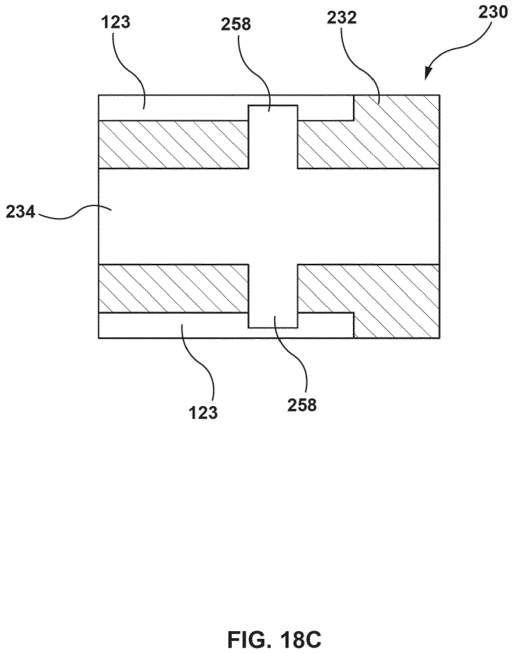

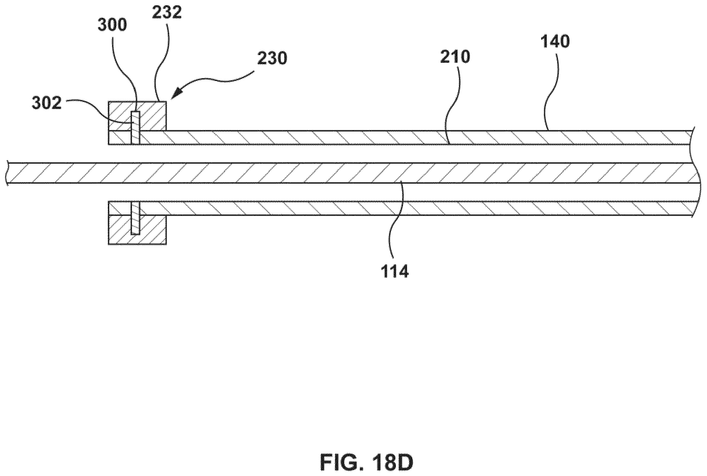

The embodiment shown in FIGS. 18A-18D further includes a cover 300. The cover 300 is similar to the cover 300 of FIGS. 16A-16D except that the cover 300 of FIGS. 18A-18B is rotatably disposed within a cover lumen 258 located within the body 232 of the capture device 230, as shown in FIG. 18D. As previously described, the cover 300 includes a body 302, a central longitudinal opening or passage 304 extending through the center of the body 302, a plurality of slot openings 306A extending through body 302 and evenly distributed radially around central longitudinal opening 304. The plurality of slot openings 306 further define the edges of a plurality of slot covers 308 evenly radiating outward from central longitudinal opening 304 and formed from the portion of body 302 occupying the space between each slot opening 306. As illustrated in FIGS. 18A-18D, the body 302 is rotatably disposed within the cover lumen 258 of the body 232 and is centered within the body 232 such that central longitudinal opening 304 of the cover 300 is aligned with central longitudinal opening 234 of the body 232. As shown in FIG. 18C, the cover 300 may be operatively coupled to the control shaft 210 such that rotation of the control shaft 210 rotates the body 302 relative to the body 232 within cover lumen 258 of the body 232.

The cover 300 is configured according to two distinct positions relative to the body 232 of the capture device 230. FIG. 18A illustrates the cover 300 in a first position, and

FIG. 18B illustrates the cover 300 in a second position. In the first position, as illustrated in FIG. 18A, each slot cover 308 covers a corresponding slot 123 at a location between the slot opening 1124 and the first end 123A of the slot 123. Each slot cover 308 further maintains a corresponding spring 254 in a compressed state. In the second position, as illustrated in FIG. 18B, each slot opening 306 over the cover 300 aligns with a corresponding slot 123.

When coupling the prosthetic valve device 150 to the capture device 230 of FIGS. 18A-18D, the springs 254 are compressed and the cover 300 is in the first position of FIG. 18A such the slot covers 308 cover the corresponding slots 123. Each capture loop 225 of the plurality of capture loops 225 is looped through a corresponding attachment member 157, 159 of the prosthetic valve device 150, as described above. The enlarged end 259 of the capture loop 225 is pushed through the flexible flap 252 at the slot opening 1124 of the slot 123 (*e.g.,* by a user, during assembly). According to embodiments herein, the flexible flap 252 has sufficient force to retain the enlarged end 259 of the capture loop 225 within the slot 123. Thus, the enlarged end 259 of each capture loop 259 is disposed within the slot 123 between the slot opening 1124 at the first end 231 of the body 232 and the slot cover 308 of the cover 300, with each spring 254 compressed on the opposite side of the slot cover 308 as each enlarged end 259. According to this embodiment, since the flexible flap 252 has sufficient force to retain the capture loop 225 within the slot 123, the capture loops 225 may be individually looped through the prosthetic valve device 150 (*e.g.,* through the first attachment members 157 and/or the second attachment members 159) and into the slots 123. Since the plurality of flexible flaps 252 keep the respective capture loops 225 in the respective slots 123, the capture loops 225 do not need to be loaded together.

When the prosthetic valve device 150 is ready to be released from the capture device 230, the cover 300 is rotated relative to the body 232, such as by the control shaft 210, thereby aligning the slot openings 306 with the corresponding slots 123. The springs 254 are thus released such that the springs 254 decompress, pushing the corresponding pushers 256 towards the corresponding slot openings 1124. The pushers 256 push the corresponding enlarged ends 259 to toward the corresponding slot openings 1124. The force of each spring 254 decompressing is sufficient to overcome the force of the corresponding flap 252, thereby pushing the corresponding enlarged end 259 out of the slot opening 1124. With the enlarged ends 259 of the capture loops 225 released from the slots 123, the prosthetic valve device 150 is released from the capture loops 225.

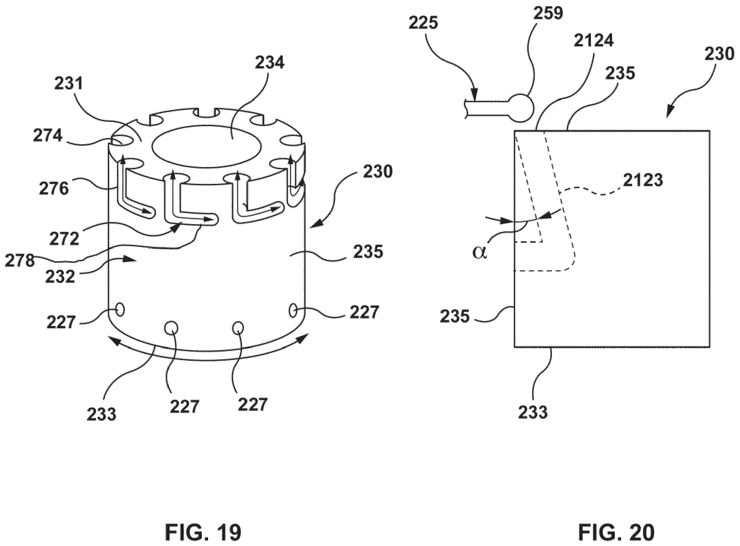

FIG. 19 shows another embodiment of a capture device 230 according to embodiments herein. The capture loops 225 are not shown in FIG. 19 for clarity but may be the capture loops 225 described with respect to FIGS. 14 and 15. The capture device 230 of FIG. 19 includes the body 232 and the central passage 234 as described above. However, the embodiment of FIG. 19 includes a plurality of shaped slots 272 distributed around the body 232 of the capture device 230. The shaped slots 272 are configured such that rotation of the body 232 releases the enlarged ends 259 of the capture loops 225. In the embodiment of FIG. 19, the shaped slots 272 are L-shaped, but this is not meant to be limiting, and other shapes the permit retention/release of the ends 259 of the capture loops 225 via rotation of the body 232 are also contemplated. Thus, the shaped slots 272 include a slot opening 274 at the first end surface 231 of the body 232, the shaped slots 272 extend longitudinally along a longitudinal portion 276 from the first end surface 231 towards the second end surface 233 (e.g., generally parallel to a central longitudinal axis of the body 232), and turn to extend generally circumferentially along a circumferential portion 278 around the outer surface 235 of the body 232. The embodiment of FIG. 19 further includes capture loop holes 277 through the body 232 as described above.

When coupling the prosthetic valve device 150 to the capture device 230 of FIG. 19, the capture loops 225 are threaded through a portion of the prosthetic valve device 150, such as the first or second attachment members 157, 159. The enlarged ends 259 of the capture loops 225 are inserted into the slot openings 1124 at the first end surface 231. The enlarged ends 259 are slid longitudinally and then circumferentially within the corresponding slots 272 to retain the enlarged ends 259 within the slots 272. When it is desired to release the prosthetic valve device 150 from the capture device 230, the body is rotated such that the enlarged ends 259 are pulled along the circumferential portions of the slots 272. The body 232 may be rotated by the control shaft 210 or the middle member 140, as explained above. Once the enlarged ends 259 are aligned with the longitudinal portion of the slots 272, the enlarged ends 259 may exit the slots 272 through the slot openings 1124 via expansion of the prosthetic valve device 150 or self-expansion of the capture loops 225.

FIG. 20 shows a capture device 230 with angled slots 2123. The angled slots 2123 are angled inwardly towards the central longitudinal axis of the body 232 from the first end surface 231 towards the second end surface 233. According to the embodiment shown in FIG. 20, each angled slot 2123 is angled at angle α. The angle α may be in the range of about 0 to about 60 degrees. The angled slots 2123 urge the exit of corresponding capture loops 225 end from the angled slot 2123 once the corresponding angled slot opening 2124 is exposed by a cover 300. The angled slots 2123 may be used with any of the embodiments described herein.

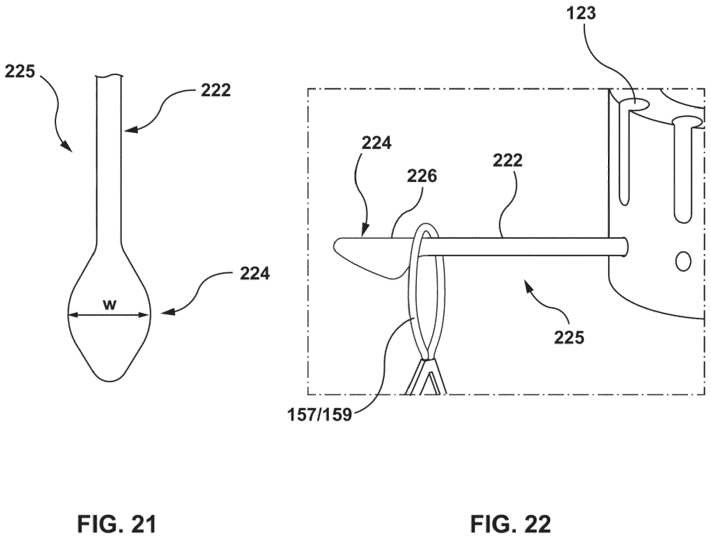

The enlarged ends 259 of the capture loops 225 may be ball shaped as shown throughout. However, other embodiments are also contemplated. For example, and not by way of limitation, FIGS. 21 and 22 show an embodiment of an enlarged end 224 to prevent release of the capture loops 225 until desired and to avoid hang-ups of the enlarged ends 259 with the attachment members 157, 159 of the prosthetic valve device 150. The enlarged end 224 shown in FIG@. 21-22 may also avoid hang-ups of the enlarged ends 224 within the slots 123. FIGS. 21-22 show a capture loop 225 with an elongated portion 222 and an enlarged portion 224. According to embodiments herein, the elongated portion 222 includes a uniform thickness along a longitudinal direction, and such thickness is suitably sized to sit loosely within the slot 123 of the capture device 230. As shown in FIG. 21, enlarged portion 224 is bulb-shaped and gradually becomes wider than the elongated portion 222. The enlarged portion 224 includes a width that is larger than the slits 1123 in the body 232 of the capture devices 230 such that the enlarged portion 224 is unable to exit the slots 123 through the slits 1123. The slot openings 1124 of the capture devices 230, on the other hand, are suitably sized to allow the enlarged portion 224 to pass therethrough. However, as can be seen in the side view of FIG. 22, a surface 226 of the enlarged portion 224 (top surface in FIG. 22) is continuous with the corresponding surface of the elongated portion 222. In other words, the surface 226 is flat with respect to the corresponding surface of the elongated portion 222. In other words, in the side view of FIG. 22, the surface 226 does not extend upwardly with respect to the corresponding surface of the elongated portion 222. A ball shaped enlarged portion, on the other hand, would extend upwardly in the view of FIG. 22. This flat surface enables the enlarged portion 224 to exit the attachments members 157/159 smoothly to release the prosthetic valve device from the capture loops 225.

While not shown in FIGS. 21 and 22, the capture loops 225 may be further treated (*e.g.,* with a chemical treatment, mechanical treatment, coating *etc*.) for a smooth surface finish to reduce friction between the capture loops 225 and an attachment member (*e.g*., first attachment member 157 or second attachment member 159) of the prosthetic valve device 150 and/or between the capture loops 225 and the slots 123 of the captured devices. In a non-limiting example, the capture loops 225 may be made of a nickel titanium alloy (Niti or Nitinol) and treated for a smooth surface finish (*e.g.,* by electropolishing).

Further disclosed herein is a delivery system. The delivery system includes an outer shaft including a capsule at a distal portion thereof configured to restrain a prosthetic device, an inner shaft disposed within the outer shaft, and a capture device disposed between the outer shaft and the inner shaft. The capture device is configured to retain a portion of the prosthetic device. The capture device includes: a body including a central passage, a plurality of radial openings extending from the central passage radially outward to an outer surface of the body, and a plurality of slots in the outer surface of the body; and a plurality of capture loops, each capture loop extending radially outward through a corresponding radial opening, each capture loop configured to extend through a portion of the prosthetic device, and a distal end of each capture loop configured to be releasably restrained in a corresponding slot of the plurality of slots.

The foregoing description has been presented for purposes of illustration and enablement and is not intended to be exhaustive or to limit the invention to the precise form disclosed. Other modifications and variations are possible in light of the above teachings. The embodiments and examples were chosen and described in order to best explain the principles of the invention and its practical application and to thereby enable others skilled in the art to best utilize the invention in various embodiments and various modifications as are suited to the particular use contemplated. It is intended that the appended claims be construed to include other alternative embodiments of the invention.

Further disclosed herein is the subject-matter of the following clauses:
1. A delivery system comprising:
   an outer shaft including a capsule at a distal portion thereof, the capsule configured to restrain a prosthetic valve device therein;
   an inner shaft disposed within the outer shaft; and
   a first capture device disposed between the outer shaft and the inner shaft, wherein the first capture device is configured to retain a portion of the prosthetic valve device, the first capture device comprising:
      a body including a central passage, a plurality of radial openings extending from the central passage radially outward to an outer surface of the body, and a plurality of slots in the outer surface of the body; and
      a plurality of capture loops, each capture loop extending radially outward through a corresponding radial opening, each capture loop configured to extend through a portion of the prosthetic valve device, and a distal end of each capture loop configured to be releasably restrained in a corresponding slot of the plurality of slots.
2. The delivery system of clause 1, further comprising a middle shaft, wherein the first capture device is coupled to the middle shaft.
3. The delivery system of clause 1 or 2, wherein the first capture device further comprises a cover, the cover being rotatable between a first position in which the cover prevents the capture loops from escaping the slots and a second position in which the cover does not prevent at least some of the capture loops from escaping the slots.
4. The delivery system of clause 3, wherein the cover includes at least one slot opening and at least one slot cover, wherein the cover is configured such that in the first position the at least one slot cover is aligned with a first slot of the plurality of slots and in the second position the at least one slot opening is aligned with the first slot of the plurality of slots.
5. The delivery system of clause 4, wherein the body of the first capture device includes a first quantity of slots, the cover includes a second quantity of slot openings, and the cover includes a third quantity of slot covers.
6. The delivery system of clause 5, wherein the first quantity, the second quantity, and the third quantity are equal such that in the first position each of the slot covers is aligned with a corresponding slot of the plurality of slots and in the second position each of the slot openings is aligned with a corresponding slot of the plurality of slots.
7. The delivery system of clause 5, wherein the second quantity is one and the third quantity is one such that in the first position the slot cover is aligned with all of slots of the plurality of slots and in the second position the slot opening is aligned with a first one of the plurality of slots and the slot cover is aligned with all of the plurality of slots except the first one.
8. The delivery system of clause 7, wherein the cover includes additional positions such that in each of the additional positions the slot opening is aligned with a different one of the plurality of slots.
9. The delivery system of clause 5, wherein the second quantity is less than the first quantity such that in the first position the slot covers are aligned with all of the slots and in the second position the slot openings are aligned with a plurality of the slots that is fewer than the first quantity, wherein cover includes additional positions such that in each of the additional positions the slot openings are aligned with different slots of the plurality of slots.
10. The delivery system of clause 1 or of any preceding clause, wherein the first capture device further comprises:
   a cover lumen extending radially from the central passage, wherein the cover lumen intersects with each of the plurality of slots between a first end of the slot and a second end of the slot;
   a cover disposed within the cover lumen and being rotatable relative to the body within the cover lumen, the cover including a plurality of slot openings and a plurality of slot covers;
   a plurality of springs, each spring of the plurality of springs coupled to the first end of a corresponding slot of the plurality of slots; and
   a plurality of flaps, each flap disposed at the second end of a corresponding slot of the plurality of slots;
   wherein in a first position, each slot cover of the plurality of slot covers is aligned with corresponding slot of the plurality of slots, each spring is in a compressed state such that each spring is disposed between a corresponding slot cover and the first end of the corresponding slot, and the distal end of each capture loop is disposed in a corresponding slot between a corresponding slot cover and a corresponding flap at the second end of the corresponding slot; and
   wherein in second position, each slot opening of the plurality of slot openings is aligned with a slot of the plurality of slots such that each spring is released from its compressed state and decompresses, thereby pushing the distal end of the corresponding capture loop with sufficient force to push the capture loop through the corresponding flap of the plurality of flaps.
11. The delivery system of clause 1 or of any preceding clause, wherein the first capture device further comprises a cover, the cover being rotatable between a first position in which the cover prevents the capture loops from escaping the slots and a second position in which the cover does not prevent at least some of the capture loops from escaping the slots, the delivery system further comprising a control shaft coupled to the cover, wherein the control shaft is configured to rotate the cover relative to the body of the first capture device.
12. The delivery system of clause 1 or of any preceding clause, wherein the plurality of slots are L-shaped including a longitudinal portion and a circumferential portion, wherein the body is rotatable between a first position wherein the distal end of each capture loop is disposed in the circumferential portion of a corresponding slot and a second position wherein the distal end of each capture loop is aligned with the longitudinal portion of the corresponding slot.
13. The delivery system of clause 1 or of any preceding clause, further comprising a pull wire coupled to proximal ends of the plurality of capture loops, wherein the pull wire is slidable within the outer shaft such that increased tension on the pull wire tightens the capture loops and decreased tension in the pull wire creates slack in the capture loops.
14. The delivery system of clause 1 or of any preceding clause, further comprising a second capture device, wherein one of the first and second capture devices is a proximal capture device and the other of the first and second capture devices is a distal capture device, wherein the proximal capture device is configured to releasably retain a first end of the prosthetic valve device and the distal capture device is configured to releasably retain a second end of the prosthetic valve device.
15. An implant assembly comprising an assembled configuration and an unassembled configuration, the implant assembly in the assembled configuration comprising:
   a prosthetic valve device comprising a stent and a prosthetic valve coupled the stent, the prosthetic valve device comprising a first end and a second end;
   a first capture device coupled to the first end of the prosthetic valve device, the first capture device including a capture body and a plurality of capture loops extending from the capture body through the first end of the prosthetic valve device and back to the capture body such that a distal end of each capture loop is releasably coupled to the capture body; and
   a second capture device coupled to the second end of the prosthetic valve device,
   wherein the implant assembly in the assembled configuration is configured to be attached to a shaft of a delivery system; and
   wherein the prosthetic valve device is configured to be released from the first capture device and the second capture device after attachment to the shaft of the delivery system.
16. The implant assembly of clause 15, wherein the first capture device further comprises a cover, the cover being rotatable between a first position in which the cover prevents the capture loops from escaping the slots and a second position in which the cover does not prevent at least some of the capture loops from escaping the slots.
17. The implant assembly of clause 16, wherein the cover includes at least one slot opening and at least one slot cover, wherein the cover is configured such that in the first position the at least one slot cover is aligned with a first slot of the plurality of slots and in the second position the at least one slot opening is aligned with the first slot of the plurality of slots.
18. The implant assembly of clause 17, wherein the body of the capture device includes a first quantity of slots, the cover includes a second quantity of slot openings, and the cover includes a third quantity of slot covers.
19. The implant assembly of clause 18, wherein the first quantity, the second quantity, and the third quantity are equal such that in the first position each of the slot covers is aligned with a corresponding slot of the plurality of slots and in the second position each of the slot openings is aligned with a corresponding slot of the plurality of slots.
20. The implant assembly of clause 15 or any of clauses 15 to 19, wherein the first capture device further comprises:
   a cover lumen extending radially from the central passage, wherein the cover lumen intersects with each of the plurality of slots between a first end of the slot and a second end of the slot;
   a cover disposed within the cover lumen and being rotatable relative to the body within the cover lumen, the cover including a plurality of slot openings and a plurality of slot covers;
   a plurality of springs, each spring of the plurality of springs coupled to the first end of a corresponding slot of the plurality of slots; and
   a plurality of flaps, each flap disposed at the second end of a corresponding slot of the plurality of slots;
   wherein in a first position, each slot cover of the plurality of slot covers is aligned with corresponding slot of the plurality of slots, each spring is in a compressed state such that each spring is disposed between a corresponding slot cover and the first end of the corresponding slot, and the distal end of each capture loop is disposed in a corresponding slot between a corresponding slot cover and a corresponding flap at the second end of the corresponding slot; and
   wherein in a second position, each slot opening of the plurality of slot openings is aligned with a slot of the plurality of slots such that each spring is released from its compressed state and decompresses, thereby pushing the distal end of the corresponding capture loop with sufficient force to push the capture loop through the corresponding flap of the plurality of flaps.

## Claims

1. A delivery system comprising:
an outer shaft including a capsule at a distal portion thereof, the capsule configured to restrain a prosthetic valve device therein;
an inner shaft disposed within the outer shaft; and
a first capture device disposed between the outer shaft and the inner shaft, wherein the first capture device is configured to retain a portion of the prosthetic valve device, the first capture device comprising:
a body including a central passage, a plurality of radial openings extending from the central passage radially outward to an outer surface of the body, and a plurality of slots in the outer surface of the body; and
a plurality of capture loops, each capture loop extending radially outward through a corresponding radial opening, each capture loop configured to extend through a portion of the prosthetic valve device, and a distal end of each capture loop configured to be releasably restrained in a corresponding slot of the plurality of slots.

2. The delivery system of claim 1, further comprising a middle shaft, wherein the first capture device is coupled to the middle shaft.

3. The delivery system of claim 1 or 2, wherein the first capture device further comprises a cover, the cover being rotatable between a first position in which the cover prevents the capture loops from escaping the slots and a second position in which the cover does not prevent at least some of the capture loops from escaping the slots.

4. The delivery system of claim 3, wherein the cover includes at least one slot opening and at least one slot cover, wherein the cover is configured such that in the first position the at least one slot cover is aligned with a first slot of the plurality of slots and in the second position the at least one slot opening is aligned with the first slot of the plurality of slots.

5. The delivery system of claim 4, wherein the body of the first capture device includes a first quantity of slots, the cover includes a second quantity of slot openings, and the cover includes a third quantity of slot covers.

6. The delivery system of claim 5, wherein the first quantity, the second quantity, and the third quantity are equal such that in the first position each of the slot covers is aligned with a corresponding slot of the plurality of slots and in the second position each of the slot openings is aligned with a corresponding slot of the plurality of slots; and/or wherein the second quantity is less than the first quantity such that in the first position the slot covers are aligned with all of the slots and in the second position the slot openings are aligned with a plurality of the slots that is fewer than the first quantity, wherein cover includes additional positions such that in each of the additional positions the slot openings are aligned with different slots of the plurality of slots.

7. The delivery system of claim 5, wherein the second quantity is one and the third quantity is one such that in the first position the slot cover is aligned with all of slots of the plurality of slots and in the second position the slot opening is aligned with a first one of the plurality of slots and the slot cover is aligned with all of the plurality of slots except the first one; and optionally wherein the cover includes additional positions such that in each of the additional positions the slot opening is aligned with a different one of the plurality of slots.

8. The delivery system of any preceding claim, wherein the first capture device further comprises:
a cover lumen extending radially from the central passage, wherein the cover lumen intersects with each of the plurality of slots between a first end of the slot and a second end of the slot;
a cover disposed within the cover lumen and being rotatable relative to the body within the cover lumen, the cover including a plurality of slot openings and a plurality of slot covers;
a plurality of springs, each spring of the plurality of springs coupled to the first end of a corresponding slot of the plurality of slots; and
a plurality of flaps, each flap disposed at the second end of a corresponding slot of the plurality of slots;
wherein in a first position, each slot cover of the plurality of slot covers is aligned with corresponding slot of the plurality of slots, each spring is in a compressed state such that each spring is disposed between a corresponding slot cover and the first end of the corresponding slot, and the distal end of each capture loop is disposed in a corresponding slot between a corresponding slot cover and a corresponding flap at the second end of the corresponding slot; and
wherein in second position, each slot opening of the plurality of slot openings is aligned with a slot of the plurality of slots such that each spring is released from its compressed state and decompresses, thereby pushing the distal end of the corresponding capture loop with sufficient force to push the capture loop through the corresponding flap of the plurality of flaps.

9. The delivery system of any preceding claim, wherein the first capture device further comprises a cover, the cover being rotatable between a first position in which the cover prevents the capture loops from escaping the slots and a second position in which the cover does not prevent at least some of the capture loops from escaping the slots, the delivery system further comprising a control shaft coupled to the cover, wherein the control shaft is configured to rotate the cover relative to the body of the first capture device; or wherein the plurality of slots are L-shaped including a longitudinal portion and a circumferential portion, wherein the body is rotatable between a first position wherein the distal end of each capture loop is disposed in the circumferential portion of a corresponding slot and a second position wherein the distal end of each capture loop is aligned with the longitudinal portion of the corresponding slot.

10. The delivery system of any preceding claim, further comprising a pull wire coupled to proximal ends of the plurality of capture loops, wherein the pull wire is slidable within the outer shaft such that increased tension on the pull wire tightens the capture loops and decreased tension in the pull wire creates slack in the capture loops; or further comprising a second capture device, wherein one of the first and second capture devices is a proximal capture device and the other of the first and second capture devices is a distal capture device, wherein the proximal capture device is configured to releasably retain a first end of the prosthetic valve device and the distal capture device is configured to releasably retain a second end of the prosthetic valve device.

11. An implant assembly comprising an assembled configuration and an unassembled configuration, the implant assembly in the assembled configuration comprising:
a prosthetic valve device comprising a stent and a prosthetic valve coupled the stent, the prosthetic valve device comprising a first end and a second end;
a first capture device coupled to the first end of the prosthetic valve device, the first capture device including a capture body and a plurality of capture loops extending from the capture body through the first end of the prosthetic valve device and back to the capture body such that a distal end of each capture loop is releasably coupled to the capture body; and
a second capture device coupled to the second end of the prosthetic valve device,
wherein the implant assembly in the assembled configuration is configured to be attached to a shaft of a delivery system; and
wherein the prosthetic valve device is configured to be released from the first capture device and the second capture device after attachment to the shaft of the delivery system.

12. The implant assembly of claim 11, wherein the first capture device further comprises a cover, the cover being rotatable between a first position in which the cover prevents the capture loops from escaping the slots and a second position in which the cover does not prevent at least some of the capture loops from escaping the slots.

13. The implant assembly of claim 12, wherein the cover includes at least one slot opening and at least one slot cover, wherein the cover is configured such that in the first position the at least one slot cover is aligned with a first slot of the plurality of slots and in the second position the at least one slot opening is aligned with the first slot of the plurality of slots.

14. The implant assembly of claim 13, wherein the body of the capture device includes a first quantity of slots, the cover includes a second quantity of slot openings, and the cover includes a third quantity of slot covers; and optionally wherein the first quantity, the second quantity, and the third quantity are equal such that in the first position each of the slot covers is aligned with a corresponding slot of the plurality of slots and in the second position each of the slot openings is aligned with a corresponding slot of the plurality of slots.

15. The implant assembly of any one of claims 11 to 14, wherein the first capture device further comprises:
a cover lumen extending radially from the central passage, wherein the cover lumen intersects with each of the plurality of slots between a first end of the slot and a second end of the slot;
a cover disposed within the cover lumen and being rotatable relative to the body within the cover lumen, the cover including a plurality of slot openings and a plurality of slot covers;
a plurality of springs, each spring of the plurality of springs coupled to the first end of a corresponding slot of the plurality of slots; and
a plurality of flaps, each flap disposed at the second end of a corresponding slot of the plurality of slots;
wherein in a first position, each slot cover of the plurality of slot covers is aligned with corresponding slot of the plurality of slots, each spring is in a compressed state such that each spring is disposed between a corresponding slot cover and the first end of the corresponding slot, and the distal end of each capture loop is disposed in a corresponding slot between a corresponding slot cover and a corresponding flap at the second end of the corresponding slot; and
wherein in a second position, each slot opening of the plurality of slot openings is aligned with a slot of the plurality of slots such that each spring is released from its compressed state and decompresses, thereby pushing the distal end of the corresponding capture loop with sufficient force to push the capture loop through the corresponding flap of the plurality of flaps.
